# EUROPEAN PATENT SPECIFICATION

(11) **EP 1 261 608 B1**
(45) Date of publication and mention of the grant of the patent: **28.04.2004**
(21) Application number: 00981112.6
(22) Date of filing: 29.11.2000
(51) Int. Cl.: C07D 471/04, A61K 31/495

(54) **DIKETOPIPERAZINE DERIVATIVES TO INHIBIT THROMBIN**
DIKETOPIPERAZINDERIVATE ALS THROMBININHIBITOREN
DERIVES DE DICETOPIPERAZINE SERVANT A INHIBER LA THROMBINE

(30) Priority: 30.11.1999 US 167901 P; 04.04.2000 US 194366 P
(43) Date of publication of application: 04.12.2002
(73) Proprietor: Best Express Worldwide Limited TrustNet Chambers, Town Road Tortola (VG)
(72) Inventor: CHENG, Yudu, Pointe-Claire, Québec H9R 5B3 (CA); MANWELL, Jeffrey, Nepean, Ontario K2E 5E8 (CA)
(74) Representative: Schrell, Andreas, Dr.
(86) International application number: PCT/CA2000/001414
(87) International publication number: WO 2001/040224

(56) References cited:
- US-A- 5 902 812
- CODY W L ET AL: "The Design of Potent and Selective Inhibitors of Thrombin Utilizing a Piperazinedione Template: Part 1" BIOORGANIC & MEDICINAL CHEMISTRY LETTERS,GB,OXFORD, vol. 9, no. 17, 6 September 1999 (1999-09-06), pages 2497-2502, XP004188851 ISSN: 0960-894X
- CODY W L ET AL: "The Design of Potent and Selective Inhibitors of Thrombin Utilizing a Piperazinedione Template: Part 2" BIOORGANIC & MEDICINAL CHEMISTRY LETTERS,GB,OXFORD, vol. 9, no. 17, 6 September 1999 (1999-09-06), pages 2503-2508, XP004188852 ISSN: 0960-894X

## Description

### BACKGROUND OF THE INVENTION

### (a) Field of the Invention

The present invention relates to compounds to inhibit blood coagulation, and more particularly to diketopiperazine derivatives, pharmaceutically acceptable salts and compositions thereof, to specifically inhibit thrombin.

### (b) Description of Prior Art

Thrombotic disorders are characterized by the formation of a thrombus obstructing the vascular blood flow, causing arterial or venous thrombosis or thromboembolism. Thrombi are composed of fibrin, platelets, white blood cells (WBCs) and red blood cells (RBCs). Thrombus formation involves several genetic and environmental factors. Genetically impaired anticoagulant mechanisms include factor V resistance to activated protein C, hyperhomocysteinemia, protein C deficiency, protein S deficiency, antithrombin deficiency and defective fibrinolysis, while thrombotic stimuli include surgery, pregnancy, oral contraceptive use and antiphospholipid antibodies. Chronic and acute thrombotic complications, including venous and arterial thrombosis, atrial fibrillation, stroke, myocardial infarction and pulmonary embolism are the leading cause of deaths worldwide.

Antithrombotic therapy involves thrombolytic drug therapy, to remove thrombi, and the use of antiplatelet drugs and anticoagulants, to inhibit coagulation. Subsequent therapy varies depending on the venous or arterial circulatory system involved and the size and location of the vessels.

The anticoagulant drugs currently used show several disadvantages (*Exp. Opin. Inves. Drugs* 1997, 6:1591-1622; *Current Pharmaceutical Design* 1995, 1:441-468; *Circulation* 1994, 90:1522-1536).

For example, heparin is the first agent to be administered parenterally in situations requiring acute anticoagulation. Heparin consists of a mucopolysaccharide of animal origin. Standard or high molecular weight heparins (HMWHs) consist of molecules of many different sizes, while depolymerized or low molecular weight heparins (LMWHs) have a molecular weight between 4000 and 6000 D.

There are several disadvantages related to the use of heparin, namely (1) heparin is a parenteral agent requiring intravenous (i.v.) or sub-cutaneous (s.c.) administration; (2) the anticoagulant dose-response curve for heparin is not linear, and *ex vivo* coagulation parameters (APTT) must be followed to monitor the degree of anticoagulation; (3) heparin is ineffective at inhibiting clot-bound thrombin; and (4) there are reports of a "rebound" reactivation of unstable angina subsequent to discontinuation of heparin therapy, and heparin has been associated with thrombocytopenia, requiring monitoring of platelet counts. Heparin-induced thrombocytopenia (HIT) is an immunoglobulin-mediated adverse drug reaction that is characterized by platelet activation, thrombocytopenia, and a high risk of thrombotic complications among patients receiving or who have recently received heparin.

In the case of venous thrombosis or pulmonary embolism, a 7-10 day course of parenteral heparin is usually followed by prolonged administration of the only currently available oral anticoagulant drug, warfarin, to prolong treatment of thrombotic complications. Heparin is generally coadministered with warfarin for a few days prior to cessation of heparin therapy.

Warfarin has several disadvantages (*The Annals of Pharmacotherapy* 1995, 29:1274-1282; *Clin. Pharmacokinet*. 1996, 30:416-444), namely: (1) it carries a risk of bleeding, (2) it exhibits adverse drug and diet interactions, and (3) it requires frequent monitoring.

Both heparin and warfarin are indirect anticoagulants and their functions depend on the presence of antithrombin and vitamin K, respectively. Consequently, following the cessation of warfarin treatment, one has to wait for the resynthesis of vitamin K-dependent coagulation factors by the liver to restore the haemostatic balance. These drawbacks limit the physician acceptance and usage of warfarin in treating thrombotic disorders.

The liabilities of the conventional anticoagulant therapy have prompted the development of novel anticoagulants over the last two decades. LMWHs were discovered to have similar efficacy to the heparin in 1976. Their favorable pharmacokinetic profiles and risk/ratios have led to widespread use in Europe since 1992 and, more recently, approval for their use in USA.

The new anticoagulant strategy is based on direct inhibition of critical enzymes in the coagulation cascade. As the final enzyme in the coagulation cascade, thrombin has been an extensively tested target. Thrombin, the key regulator of the thrombotic process, is a trypsin-like serine protease. Thrombin has many and varied biological functions, but its main action is to catalyze the transformation of fibrinogen to fibrin, whether the thrombin is soluble in plasma or fibrin-bound. Fibrin is then polymerized and cross-linked by the action of activated blood factor XIII to form insoluble blood clotting. Thrombin also activate blood factors V and VIII which in turn accelerates the blood coagulation by a feed-back mechanism. As a potent activator of platelets, thrombin also plays an important role in driving the growth of platelet-rich thrombi in the arterial circulation. The fibrin deposition and platelet aggregation can thus be interrupted when thrombin is inhibited. However, thrombin is similar to numerous serine-proteases present in the human body and particularly in the blood, such as plasmin. A thrombin inhibitor therefore needs to be specific to thrombin.

Thrombin inhibitors can directly inactivate thrombin by binding to thrombin active site and/or fibrinogen recognition exosite (FRE), whereby fibrinogen is transformed into fibrin. For example, hirudin is a naturally occurring 63-amino acid anticoagulant which is produced in the salivary glands of the blood sucking leech *Hirudo medicinalis.* Hirudin inhibits thrombin by directly binding both the thrombin active site and the FRE with an inhibition constant (Kᵢ) value of 2.0 x 10⁻¹⁵M against thrombin *(Biochemistry* 1986, 25:4622-4628). Hirugen is a peptide derived from the anionic carboxy-terminus of hirudin and binds only the FRE of thrombin with a Kᵢ value of 1.44 x 10⁻⁷M (*J. Biol. Chem.* 1989, 264:8692-8698). Hirulogs or Bivalirudin is a synthetic peptide consisting of a hirugen-like FRE-binding sequence linked by a glycine-spacer to the substrate-like active-site binding moiety, D-phenyalanine-prolyl-arginine with a Kᵢ value of 2.3 x 10⁻⁹M.

Except for a slightly better safety profile in terms of the bleeding complications, the above-mentioned direct thrombin inhibitors have shown no better and even worse features than heparin, namely (1) relatively short half lives, (2) parenteral administration, and (3) cost-ineffectiveness.

U.S. Patent Nos. 4,258,192 and 4,201,863 disclose a synthetic small molecule thrombin inhibitor with a Kᵢ value of 1.9 x 10⁻¹⁰M for human thrombin, which is commercialized as Argatroban (Novastan, Mitsubishi Chemical Corp *Cardiovasc. Drug Rev.* 1991, 9:247-263). It was developed for the indications of chronic arterial obstruction, acute ischaemic stroke and haemodialysis in antithrombin III (ATIII)-deficient patients, and as a replacement for heparin in patients at risk of (HIT). However, Argatroban is still not an ideal small molecule thrombin inhibitor due to the following problems: it is (1) not orally bioavailable; (2) less effective on venous than arterial thrombosis; (3) possibly dose-dependent; (4) thrombin rebound effect; (5) not more effective than heparin in treatment of unstable angina, coronary angioplasty and acute myocardial infarction.

It would therefore be highly desirable to be provided with an orally available thrombin inhibitor. The interest in orally bioavailable thrombin inhibitors is high (*Am. J. Cardiol.* 1995, 75:27B-33B). A small molecule thrombin active site inhibitor that would selectively and reversibly inhibit thrombin would present a distinct advantage over warfarin with respect to side-effects and monitoring, as described above. The selectivity of a thrombin inhibitor compared to warfarin would allow it to be used with relative safety in both arterial and venous thrombosis. Another distinct advantage of a small molecule thrombin inhibitor would be its potentially important ability to inhibit clot-bound thrombin as well as fluid-phase thrombin.

### SUMMARY OF THE INVENTION

One aim of the present invention is to provide an orally available specific thrombin inhibitor.

In accordance with the present invention there is provided a compound of the following formula I: 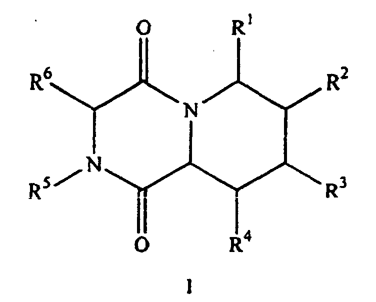 or a pharmaceutically acceptable salt or stereoisomer thereof, wherein R¹, R² and R⁴ consist of a hydrogen, alkyl or aryl moiety, R³ consist of an alkyl or aryl moiety, R⁵ consists of a hydrogen, alkyl, aryl, hydroaryl, heteroaryl, hydroheteroaryl, sulfonylalkyl, sulfonylaryl, sulfonylhydroaryl, sulfonylheteroaryl or sulfonylhydroheteroaryl moiety, and R⁶ consists of a hydrogen, alkyl, aryl, hydroaryl, heteroaryl or hydroheteroaryl moiety. Such a compound inhibits thrombin or blood coagulation and may be used as an antithrombotic or an anticoagulant.

For example, R⁵ may consist of an alkyl, aryl, hydroaryl, heteroaryl or hydroheteroaryl moiety. More particularly, R¹, R² and R⁴ may consist of a hydrogen moiety, R³ may consist of a methyl moiety, R⁵ may consist of 1,2,3,4-tetrahydra-3-methyl-8-quinolinesulfonyl, and R⁶ may consist of 3-guanidinopropyl. Such a compound has a high inhibition constant (Kᵢ is 5.3 x 10⁻¹⁵M).

In accordance with the present invention, there is further provided a pharmaceutical composition comprising such a compound as an active ingredient, in association with a pharmaceutically acceptable carrier. The pharmaceutical composition may be suitable for oral administration. The active ingredient may be used in a composition such as a tablet, capsule, solution or suspension containing about 5 to about 500 mg per unit of dosage of a compound of formula I or a mixture thereof. The compounds may be combined in a conventional manner with a physiologically acceptable vehicle or carrier including suitable expedients, binders, preservatives, stabilizers, flavors, etc. as accepted in the pharmaceutical practice.

In accordance with the present invention, there is further provided a method for substantially preventing thrombin activity in a mammal or a human or a tissue thereof. The method comprises administering an effective amount of such the compound or the pharmaceutical composition to the mammal, human or tissue.

In accordance with the present invention, there is further provided a method for treating a coagulation disorder in a mammal or a human or a tissue thereof. The method comprises administering an effective amount of the compound or the pharmaceutical composition to the mammal, human or tissue. Examples of coagulation disorders include thrombosis or heparin-induced thrombocytopenia (HIT).

The compounds of the present invention may be administered orally to various mammalians known to thrombotic disorders, such as humans, cats, dogs, monkeys, mice and the like in an effective dosage range of 0.1 to 100 mg/kg, preferably about 0.2 to 50 mg/kg and more preferably about 0.5 to 25 mg/kg on a regimen in single or 2 to 4 divided daily doses.

Although the compound of formula I of the present invention inhibits thrombin and may be used as an anticoagulant, it may also be used in combination with other antithrombotic or anticoagulant drugs.

The compound of the present invention comprises an extra ring and substitutions to a diketopiperadine structure, and is more rigid compared to known diketopiperadine derivatives.

The compound of the present invention inhibits blood coagulation by specifically binding to thrombin. Compared to anticoagulant drugs such as Heparin, Warfarin, Hirudin, Hirugen, Hirulogs and Argatroban. The compound of the present invention exhibits oral bioavailability, an increased half-life, effectiveness on venous thrombosis and limited or no "rebound" effect on thrombin, contrary to heparin. The compound of the present invention also reduces the risk of heparin-induced thrombocytopenia (HIT).

For the purpose of the present invention, the following terms are defined below.

The term "alkyl" is intended to mean a straight or a branched chain radical(s) or cyclic ring(s) of up to 18 carbons, preferably of 1 to 8 carbons, such as methyl, ethyl, propyl, butyl, pentyl, hexyl, heptyl, octyl, nonyl, decyl, undecyl, dodecyl, and the various branched chain isomers thereof and/or 1 or 2 of the following substituents: an aryl substituent (for example, to form benzyl or phenethyl), a cycloalkyl substituent, an alkylcycloalkyl substituent, an alkenyl substituent, an alkynyl substituent, hydroxy, alkoxy, halogen, amino, alkylamino, dialkylamino, guanidino or carboxy substituent.

The term "aryl" is intended to mean a monocyclic, bicyclic or tricyclic aromatic group(s) containing 6 to 14 carbons in the ring portion, such as phenyl, naphtyl or anthracenyl. The aryl moiety may include substituted aryl, which may include 1 or 2 substituents such as alkyl, cyano, amino, alkylamino, dialkylamino, nitro, carboxy, carboalkoxy, trifluoromethyl, halogen, alkoxy, arylalkoxy or hydroxy.

The term "hydroaryl" is intended to mean 10 to 14-membered aromatic rings, such as tetrahydronapthyl, tetrahydroanthracenyl and the like. Hydroaryl may include substituted hydroaryl, which may include 1 or 2 substituents such as alkyl, cyano, amino, alkylamino, dialkylamino, nitro, carboxy, carboalkoxy, trifluoromethyl, halogen, alkoxy, arylalkoxy or hyroxy.

The term "heteroaryl" is intended to mean 5- to 14-membered aromatic ring(s) which includes 1,2 or 3 heteroatoms such as nitrogen, oxygen or sulfur, such as 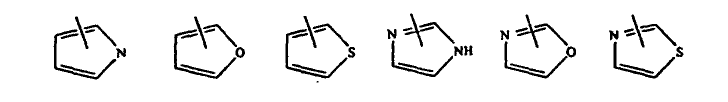 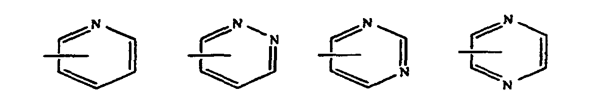 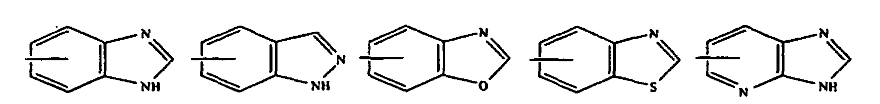 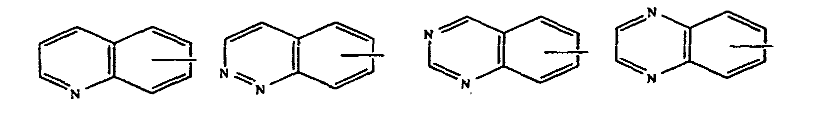 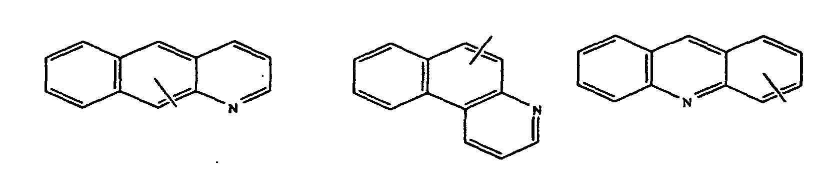 and the like. The heteroaryl rings may optionally be fused to aryl rings such as defined previously. The heteroaryl rings may include a substituted heteroaryl, which may include 1 or 2 substituents such as alkyl, cyano, amino, alkylamino, dialkylamino, nitro, carboxy, carboalkoxy, trifluoromethyl, halogen, alkoxy, arylalkoxy or hyroxy.

The term "hydroheteroaryl" is intended to mean a reduced form of the above-mentioned heteroaryl rings, such as:

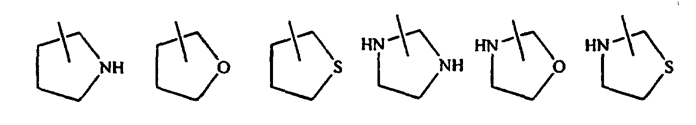

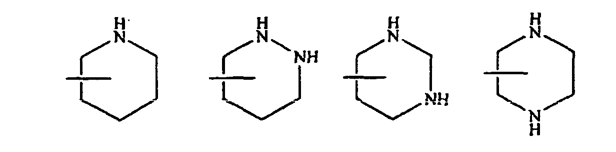

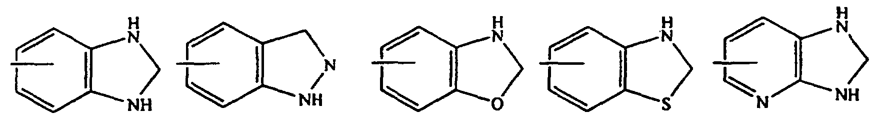

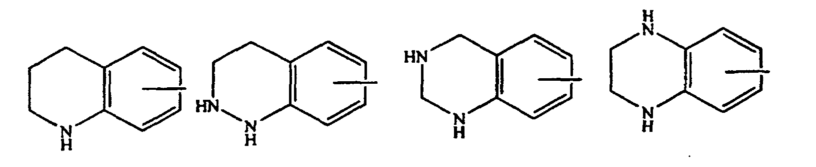

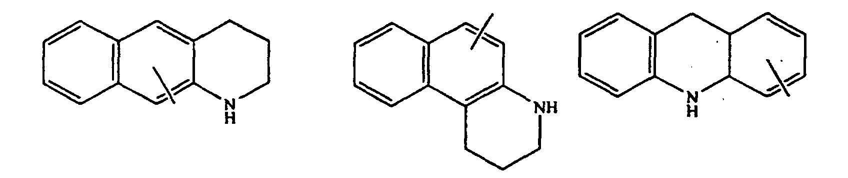

The term "sulfonylaryl" is intended to mean a sulfonyl group (SO₂) in which an aryl group such as defined previously is attached.

The term "sulfonylhydroaryl" is intended to mean a sulfonyl group (SO₂) in which an hydroaryl group such as defined previously is attached.

The term "sulfonylheteroaryl" is intended to mean a sulfonyl group (SO₂) in which a heteroaryl group such as defined previously is attached.

The term "sulfonylhydroheteroaryl" is intended to mean a sulfonyl group (SO₂) in which a hydroheteroaryl group such as defined previously is attached.

A pharmaceutically acceptable acid salt may be obtained from the compound of formula I of the present invention by reacting the free base with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like.

### BRIEF DESCRIPTION OF THE DRAWINGS

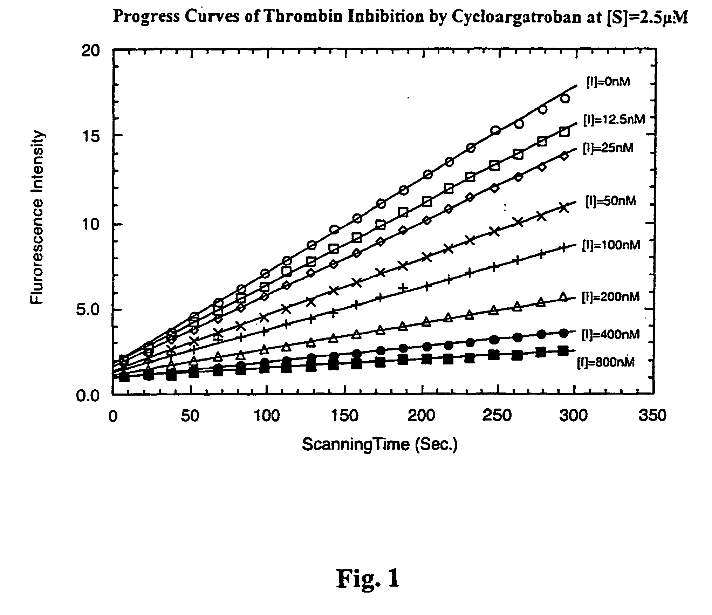

Fig. 1 illustrates a progressive curve of an *in vivo* thrombin inhibition assay at the substrate concentration ([S]) of 2.5 µM by the compound of cycloargatroban (formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl.

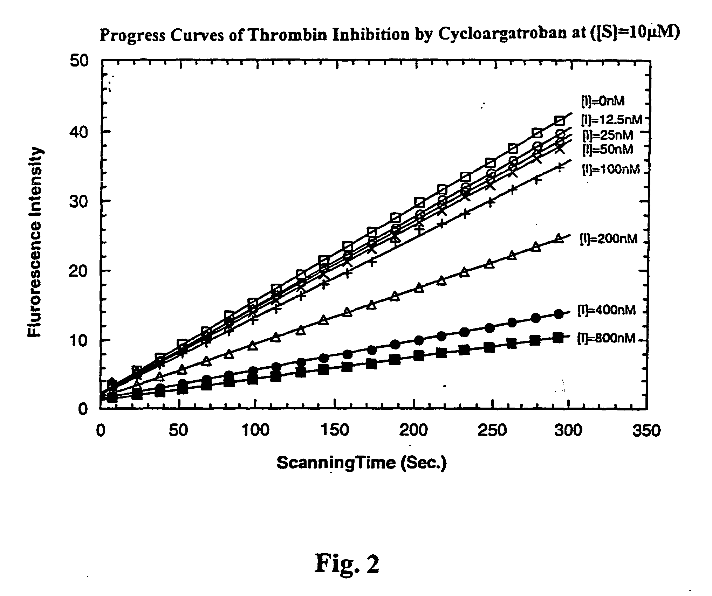

Fig. 2 illustrates a progressive curve of an in vivo thrombin inhibition assay at the substrate concentration ([S]) of 10 µM by the compound of cycloargatroban (formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl.

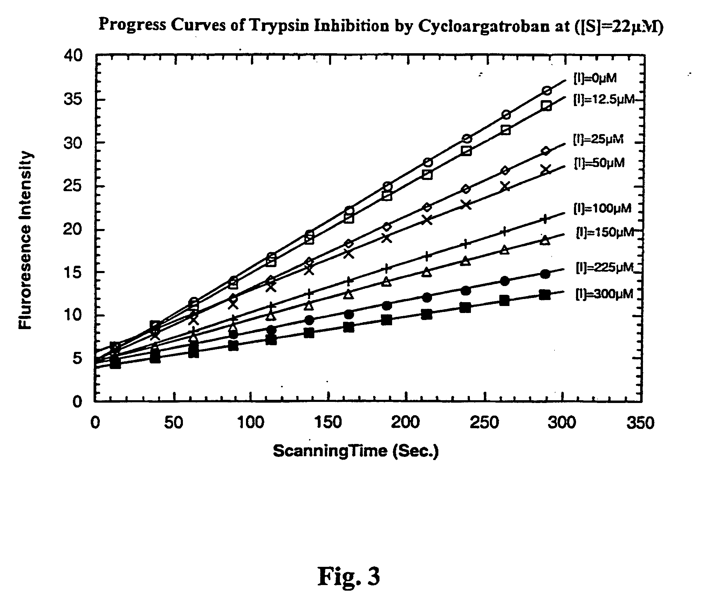

Fig. 3 illustrates a progressive curve of an *in vivo* trypsin inhibition assay at the substrate concentration ([S]) of 22 µM by the compound of cycloargatroban (formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl.

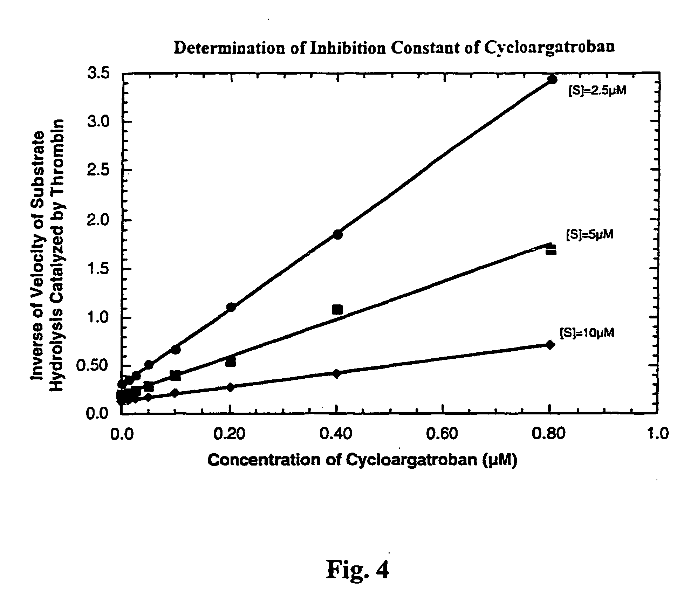

Fig. 4 illustrates an in vivo kinetic assay to determine the inhibition constant (Ki) for thrombin by the compound of cycloargatroban (formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl.

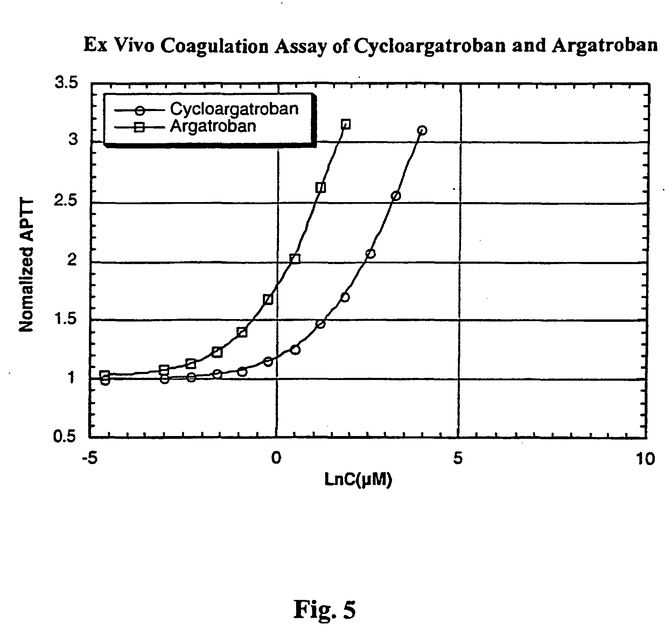

Fig. 5 illustrates an *ex vivo* coagulation assay of the compound of cycloargatroban (formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl and the reference compound of argatroban with the chemical composition shown in structure XIII below.

### DETAILED DESCRIPTION OF THE INVENTION

In accordance with the present invention, there are provided compounds which are useful as potent and specific inhibitors of thrombin and blood coagulation *in vitro* and *in vivo* in mammals.

The invention involves the preparation of diketopiperazine derivatives of the formula I:

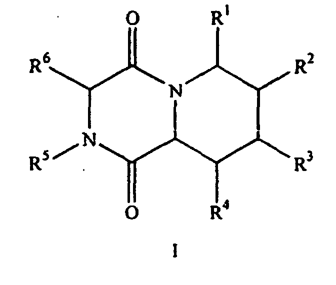

The compounds of formula I of the invention may be prepared according to the following Reaction Sequence I: 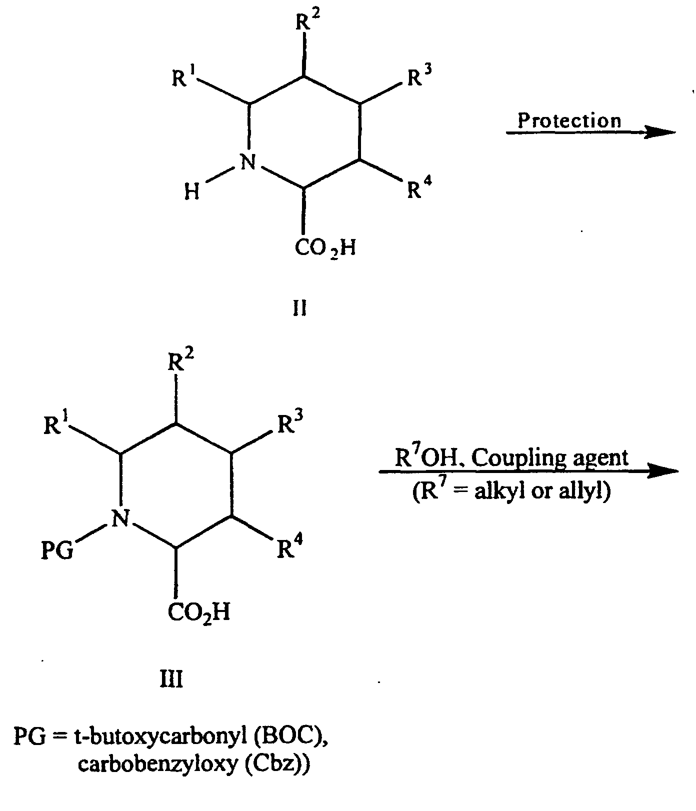 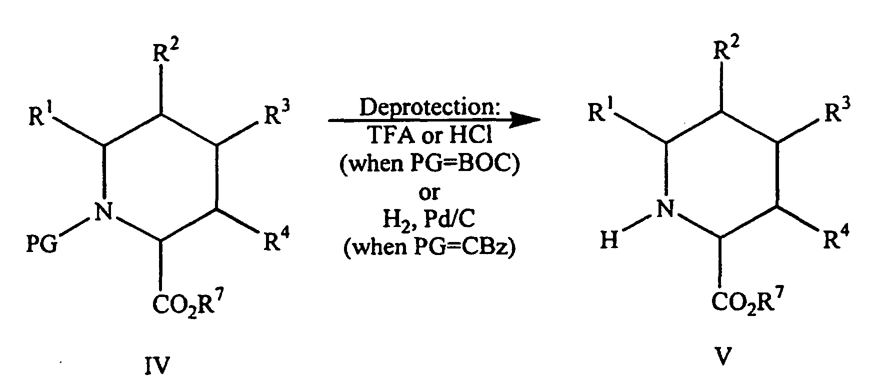 or

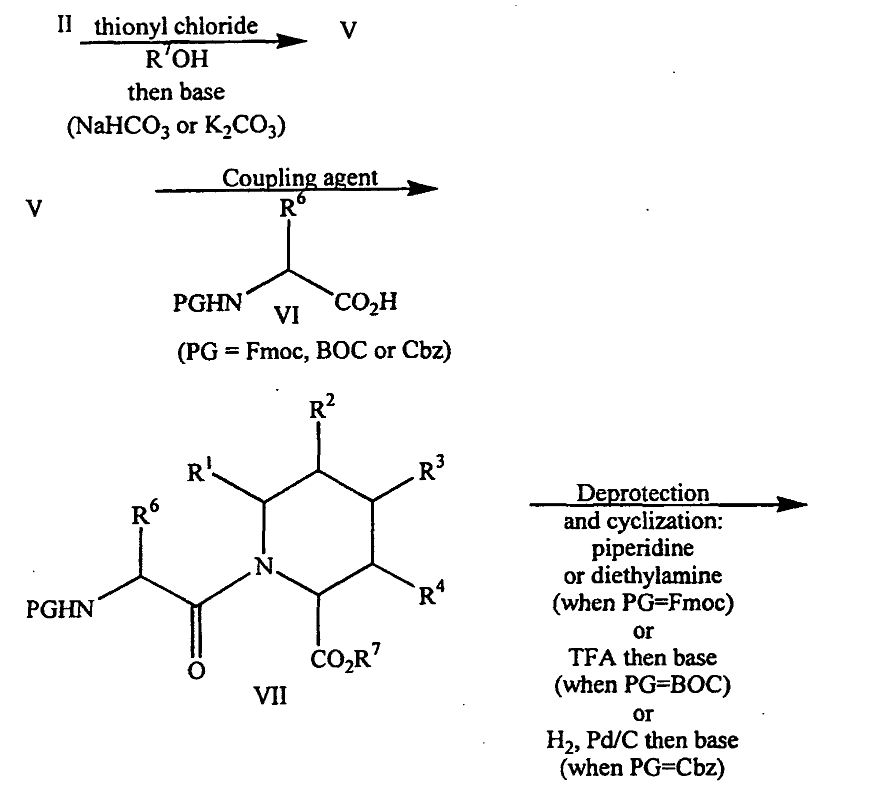

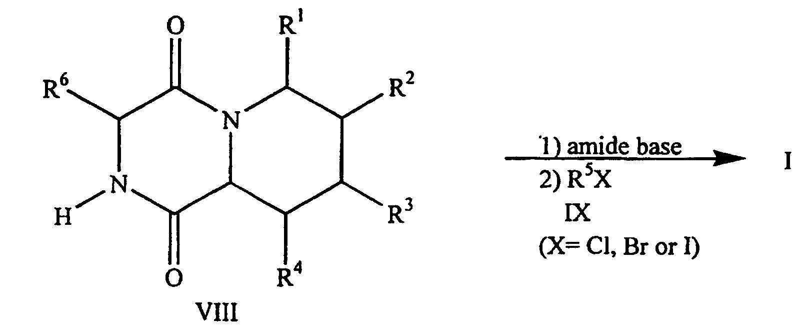

The amino acid II is protected with a tert-butoxycarbonyl group (BOC) using di-tert-butyl dicarbonate in 10% triethylamine (TEA) in methanol, or with a benzyloxycarbonyl group (Cbz) using benzyl chloroformate and aqueous sodium hydroxide solution in an organic solvent such as dioxane, tetrahydrofuran (THF) or ether. The protected amino acid III is esterified using a coupling reaction with an alcohol in the presence of dicyclohexylcarbodiimide (DCC), diisopropylcarbodiimide (DIC) or 2-(1H-benzotriazole-1-yl)-1,1,3,3-tetramethyluronium tetrafluoroborate (TBTU) and 4-dimethylaminopyridine (DMAP) or N-hydroxybenzotriazole (HOBT) and in the presence of an inert organic solvent such as dimethylformamide (DMF), N-methyl pyrrolidinone (NMP), dichloromethane (DCM) or THF at temperatures within the range of -20°C to -5°C, to form an ester IV.

The ester IV is deprotected by treatment with trifluoroacetic acid (TFA) or hydrochloric acid (HCl) in the presence of a dry inert solvent such as DCM, THF, ethyl acetate or chloroform (BOC), or by hydrogenation over palladium on carbon in an alcoholic solvent (Cbz) at ambient temperature. Alternatively, the ester V may be prepared by the addition of thionyl chloride to an alcoholic solution of amino acid II at a temperature range within 0°C to 20°C followed by neutralization with a base such as sodium bicarbonate or potassium carbonate and the like. The ester V is made to undergo a coupling reaction with a protected amino acid derivative VI in the presence of a coupling reagent such as DCC, DIC or TBTU, and DMAP or HOBT, and a tertiary organic amine base such as TEA or diisopropylethylamine (DIPEA), and in the presence of an inert organic solvent such as DMF, NMP, THF or DCM at temperatures within the range of 0°C to 20°C to form the peptide VII. The peptide VII is deprotected and cyclized in the presence of piperidine or diethylamine, and an inert organic solvent such as DMF, NMP, DCM or THF and at ambient temperature (Fmoc), or deprotected by treatment with TFA or HCl in the presence of a dry inert solvent such as DCM, THF, ethyl acetate or chloroform (BOC), or by hydrogenation over palladium on carbon in an alcoholic solvent (Cbz) at ambient temperature, followed by addition of base to cause cyclization. The diketopiperazine VIII is treated with an amide organic base such as lithium bis (trimethylsilyl)amide (LHMDS) or lithium diisopropylamide (LDA), and in dry THF solvent at 0°C, followed by the addition of an alkylating agent IX at a temperature within the range of 0°C and 20°C to form the diketopiperazine I.

The compounds of formula I of the present invention may also be prepared according to the following Reaction Sequence II:

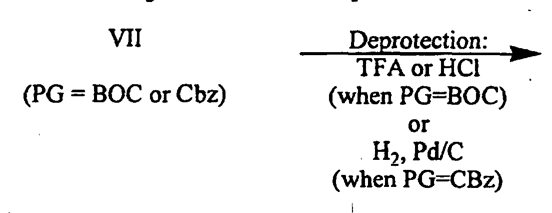

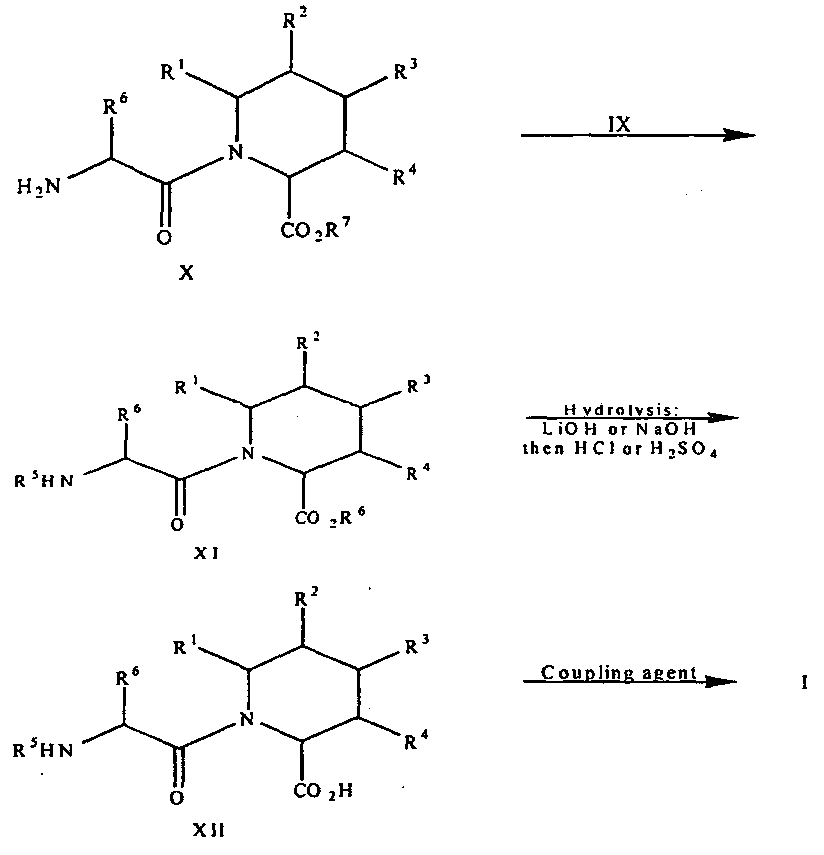

The peptide VII wherein PG is BOC or Cbz, is deprotected by treatment with TFA or HCl in the presence of a dry inert solvent such as DCM, THF, ethyl acetate or chloroform at ambient temperature (BOC), or by hydrogenation over palladium on carbon in an alcoholic solvent (Cbz). The peptide X is made to undergo a reaction with an alkylating agent IX, in the presence of a tertiary organic amine base such as pyridine, TEA or DIPEA, and in the presence of a dry inert solvent such as DCM, THF or chloroform at ambient temperature to form a peptide XI. The ester of peptide XI is hydrolyzed by treatment with an alkali metal base such as sodium hydroxide (NaOH) or lithium hydroxide (LiOH) in the presence of an alcohol solvent such as methanol or ethanol. The reaction mixture is acidified with HCl or sulfuric acid (H₂SO₄) to form an acid XII.

The acid XII is made to undergo an intramolecular cyclization reaction in the presence of TBTU, and HOBT, and DIPEA in an inert organic solvent such as DMF, NMP, THF or DCM at ambient temperature to form the diketopiperazine I.

The compounds of formula I of the present invention, wherein R⁶ is 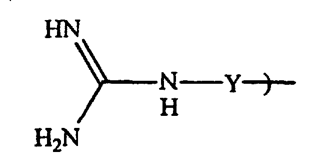 and Y is an alkyl, aryl, hydroaryl, heteroaryl or hydroheteroaryl moiety, may be prepared according to the following Reaction Sequence III:

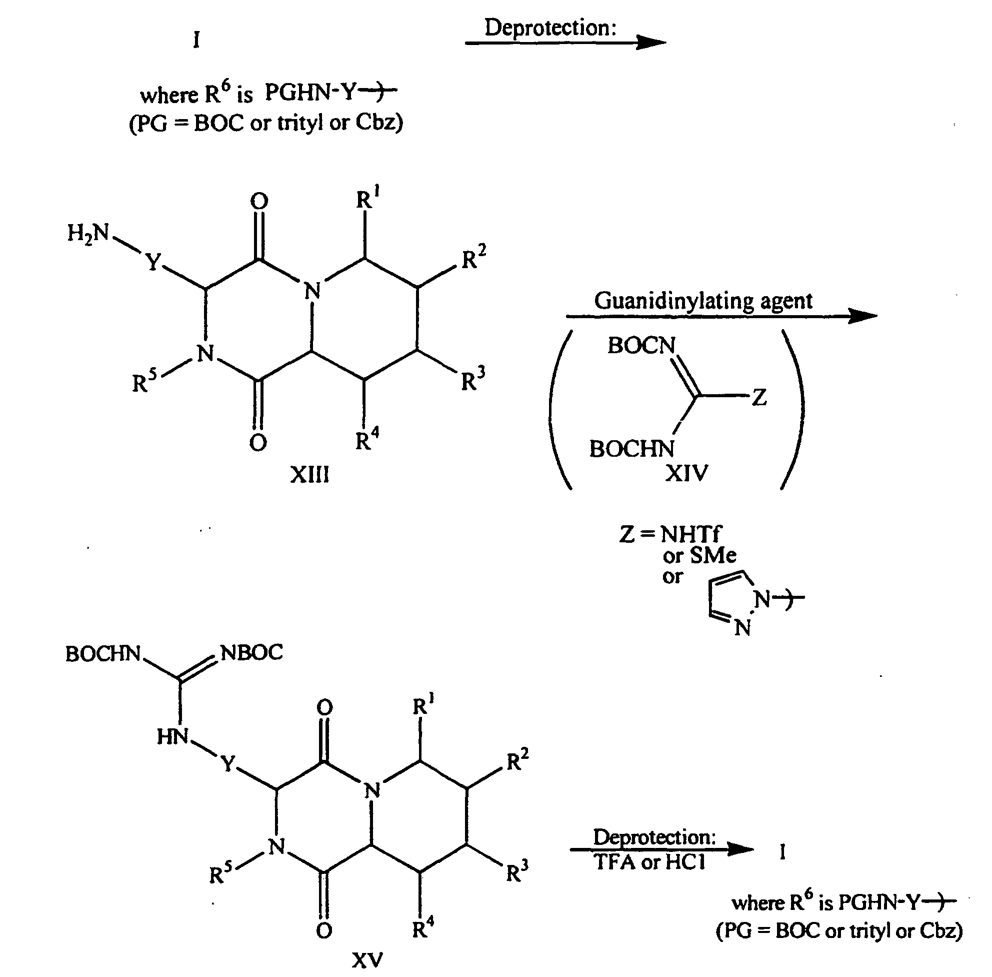

The diketopiperazine I is prepared following Reaction Sequence I or II wherein R⁶ is 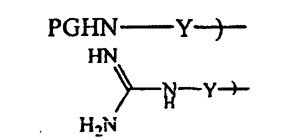 and deprotected by treatment with TFA or HCl in the presence of a dry inert solvent such as DCM, THF, ethyl acetate or chloroform (BOC, Trityl and the like), or by hydrogenation over palladium on carbon in an alcoholic solvent (Cbz) at ambient temperature. The diketopiperazine XIII is guanidinylated in the presence of guanidinylating reagents XIV such as N,N'-bis(tert-butoxycarbonyl)-N"-trifluromethanesulfonylguanidine, 1-[N,N'-bis(*tert*-butoxycarbonyl)amido]pyrazole or N,N'-bis(*tert*-butoxycarbonyl)-S-methylisothiourea, and a tertiary organic amine base such as TEA or DIPEA, and in the presence of an inert organic solvent such as DMF, NMP, THF or DCM at ambient temperature to form a protected guanidinylated diketopiperazine XV.

The diketopiperazine XV is deprotected by treatment with TFA or HCl in the presence of a dry inert solvent such as DCM, THF, ethyl acetate or chloroform at ambient temperature to form diketopiperazine I, wherein R⁶ is

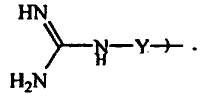

The compounds of formula I of the invention wherein R⁵ is hydroheteroaryl may be prepared according to the following Reaction Sequence IV.

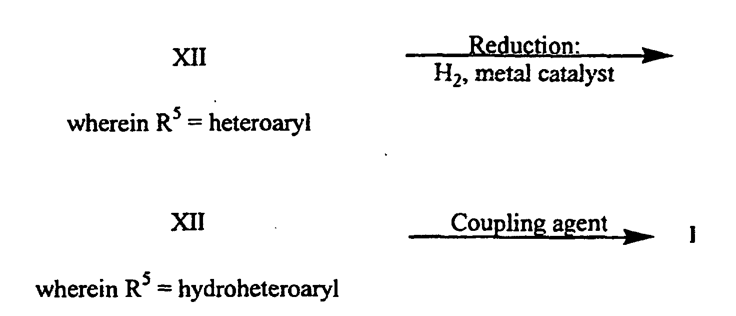

The acid XII is prepared following Reaction Sequence II wherein R⁵ is an aryl moiety and is subjected to a reduction in the presence of a catalyst containing metals such as palladium, platinum, rhodium or nickel, and at temperatures within the range of 20°C to 100°C, and pressures within the range of 1 to 100 atmospheres to form the acid XII, wherein R⁵ is a hydroheteroaryl moiety. The acid XII wherein R⁵ is hydroheteroaryl is made to undergo an intramolecular cyclization reaction in the presence of a coupling agent TBTU, and HOBT, and DIPEA, and in the presence of an inert organic solvent such as DMF, NMP, THF or DCM at ambient temperature to form the diketopiperazine I, wherein R⁵ is hydroheteroaryl.

The present invention will be more readily understood by referring to the following examples, which are given to illustrate the invention rather than to limit its scope.

### EXAMPLE I

### N-(tert-Butoxycarbonyl)-D-2-piperidinecarboxylic acid, allyl ester

N-(tert-Butoxycarbonyl)-D-2-piperidinecarboxylic acid (2.0 g, 8.7 mmol, BACHEM) was dissolved in dichloromethane (40 mL), cooled to -20°C, allyl alcohol (1.0 ml, 15.0 mmol, Aldrich), dicyclohexylcarbodiimide (1.8 g, 8.7 mmol, Aldrich) and 4-dimethylaminopyridine (0.11 g, 0.87 mmol, Aldrich) were added and the reaction mixture was stirred between -5°C and -10°C for 4 h. After filtration to remove the urea byproduct, the reaction mixture was concentrated *in vacuo*. The resulting oil was subjected to chromatography on 100 g of silica gel and eluted with 15:1 hexane/ethyl acetate to give the title compound as a clear colorless liquid (2.33 g, 99%).

### EXAMPLE 2

### (2R,4R)-N-(tert-butoxycarbonyl)-4-methyl-2-piperidinecarboxylic acid, allyl ester

(2R,4R)-4-Methyl-2-piperidinecarboxylic acid (250 mg, 1.75 mmol) was dissolved in 10% triethylamine in methanol (30 mL), cooled to 0°C and di-tert-butyl dicarbonate (0.48 mL, 2.10 mmol, Aldrich) was added. After 2 h, the reaction mixture was concentrated in *vacuo* and sodium phosphate monobasic (10 mg) was added. The residue was dissolved in 1:1 ethyl acetate/water (10 mL) and the solution was adjusted to pH 2 with 1N hydrochloric acid. The mixture was extracted with ethyl acetate (4 x 20 mL) and the combined organic extracts were dried over anhydrous sodium sulfate and concentrated *in vacuo.* The resulting white solid was dissolved in dichloromethane (8 mL) and cooled to - 20°C. Allyl alcohol (0.20 ml, 2.98 mmol, Aldrich), dicyclohexylcarbodiimide (361 mg, 1.75 mmol, Aldrich) and 4-dimethylaminopyridine (22 mg, 0.18 mmol, Aldrich) were added and the reaction mixture was stirred between -5°C and -10°C for 5 h. After filtration to remove the urea byproduct, the reaction mixture was concentrated *in vacuo*. The resulting oil was subjected to chromatography on 10 g of silica gel and eluted with 9:1 hexane/ethyl acetate to give the title compound as a clear colorless liquid (457 mg, 92%).

### EXAMPLE 3

### (3S,6R)-Bicyclo[4.4.0]-1,4-diaza-3-[(4-nitrophenyl)methyl]-4-N-(4-tert-butylbenzenesulfonyl)-2,5-decanedione, trifluroacetate salt

### 3A) 1-[N^{α}-(9-fluorenylmethoxycarbonyloxy)-L-4-nitrophenylalanyl]-D-2-piperidinecarboxylic acid, allyl ester

The pipecolic ester of Example 1 (259 mg, 0.96 mmol) was dissolved in 1:1 trifluroacetic acid/dichloromethane (5 mL) and stirred for 3 h. The reaction mixture was concentrated *in vacuo* and placed on a vacuum pump overnight. The resulting oil was dissolved in dimethylformamide (5 mL), cooled to 0°C and diisopropylethylamine (0.50 mL, 2.88 mmol, Aldrich) was added. After stirring for 5 min, N^{α}-(9-fluorenylmethoxycarbonyloxy)-L-4-nitrophenylalanine (500 mg, 1.16 mmol, Novabiochem), N-hydroxybenzotriazole (205 mg, 1.34 mmol, Novabiochem) and 2-(1H-benzotriazole-1-yl)-1,1,3,3-tetramethyluronium tetrafluoroborate (430 mg, 1.34 mmol, Novabiochem) were added. The reaction mixture was stirred for 72 h, poured into ethyl acetate (125 mL) and washed with 10% hydrochloric acid (2 x 25 mL), saturated sodium bicarbonate solution (2 x 25 mL) and brine (25 mL). The organic layer was dried over anhydrous sodium sulfate and concentrated *in vacuo*. The resulting oil was subjected to chromatography on 50 g of silica gel and eluted with 7:3 hexane/ethyl acetate to give the title compound as a white solid (457 mg, 82%).

### 3B) (3S,6R)-Bicyclo[4.4.0]-1,4-diaza-3-[(4-nitrophenyl)methyl]-2,5-decanedione

To a solution of Part 3A ester (200 mg, 0.34 mmol) in dichloromethane (68 mL) was added piperidine (1.68 mL, 17.0 mmol, Aldrich) and the reaction mixture was stirred for 1 h. The reaction mixture was concentrated in vacuo and the resulting oil was subjected to chromatography on 20 g of silica gel and eluted with 19:1 dichloromethane/methanol to give the title compound as a pale yellow solid (69 mg, 67%).

### 3C) (3S,6R)-Bicyclo[4.4.0]-1,4-diaza-3-[(4-nitrophenyl)methyl]-4-N-(4-tert-butylbenzenesulfonyl)-2,5-decanedione, trifluroacetate salt

To a solution of Part 3B diketopiperazine (20 mg, 0.066 mmol) in anhydrous tetrahydrofuran (1 mL, Aldrich) under a nitrogen atmosphere at 0°C was added 1.0 M lithium bis(trimethylsilyl)amide in tetrahydrofuran (0.090 mL, 0.090 mmol, Aldrich) and the reaction mixture was stirred for 1 h. 4-tert-Butylbenzenesulfonyl chloride (23 mg, 0.10 mmol) was added in one portion and the mixture was stirred at room temperature for 2 h. Brine (5 mL) was added and the reaction mixture was extracted with ethyl acetate (3x10 mL). The combined organic extracts were dried over anhydrous sodium sulfate and concentrated in vacuo. The resulting oil was subjected to chromatography on 25 g of silica gel and eluted with 9:1 hexane/ethyl acetate then 7:3 hexane/ethyl acetate to give the title compound as a white solid (23 mg, 70%): Mass spec. (EI): (M⁺) at 499.

### EXAMPLE 4

### (3S,6R)-Bicyclo[4.4.0]-1,4-diaza-3-(3-guanidinopropyl)-4-N-(4-tert-butylbenzenesulfonyl)-2,5-decanedione, trifluroacetate salt.

### 4A) 1-[N^{γ}-(4-Methyltrityl)-N^{α}-(9-fluorenylmethoxy carbonyloxy)-L-ornithinyl]-D-2-piperidinecarboxylic acid, allyl ester

Example 1 pipecolic ester (500 mg, 1.86 mmol) was dissolved in 1:1 trifluroacetic acid/dichloromethane (8 mL) and stirred for 3 h. The reaction mixture was concentrated in vacuo and placed on a vacuum pump overnight. The resulting oil was dissolved in dimethylformamide (8 mL), cooled to 0°C and diisopropylethylamine (0.97 mL, 5.58 mmol, Aldrich) was added. After stirring for 5 min, N^{γ}-(4-methyltrityl)-N^{α}-(9-fluorenylmethoxycarbonyloxy)-L-ornithine (1.36 g, 2.23 mmol, Novabiochem), N-hydroxybenzotriazole (398 mg, 2.60 mmol, Novabiochem) and 2-(1H-benzotriazole-1-yl)-1,1,3,3-tetramethyluronium tetrafluoroborate (835 mg, 2.60 mmol, Novabiochem) were added. The reaction mixture was stirred for 96 h, poured into ethyl acetate (125 mL) and washed with 10% hydrochloric acid (2 x 25 mL), saturated sodium bicarbonate solution (2 x 25 mL) and brine (25 mL). The organic layer was dried over anhydrous sodium sulfate and concentrated in vacuo. The resulting oil was subjected to chromatography on 75 g of silica gel and eluted with 3:1 hexane/ethyl acetate to give the title compound as a white solid (1.22 g, 86%).

### 4B) (3S,6R)-Bicyclo[4.4.0]-1,4-diaza-3-(3-N-(4-methyltrityl)aminopropyl)-2,5-decanedione

To a solution of Part 4A ester (500 mg, 0.66 mmol) in dichloromethane (132 mL) was added piperidine (3.26 mL, 33.0 mmol, Aldrich) and the reaction mixture was stirred for 3 h. The reaction mixture was concentrated *in vacuo* and the resulting oil was subjected to chromatography on 40 g of silica gel and eluted with 1:1 hexane/ethyl acetate to give the title compound as a white solid (288 mg, 91%).

### 4C) (3S,6R)-Bicyclo[4.4.6]-1,4-diaza-3-(3-N-(4-methyltrityl)aminopropyl)-4-N-(4-tert-butylbenzene sulfonyl)-2,5-decanedione

To a solution of Part 4B diketopiperazine (150 mg, 0.31 mmol) in anhydrous tetrahydrofuran (5 mL, Aldrich) under a nitrogen atmosphere at 0°C was added 1.0 M lithium bis(trimethylsilyl)amide in tetrahydrofuran (0.42 mL, 0.42 mmol, Aldrich) and the reaction mixture was stirred for 1 h. 4-tert-Butylbenzenesulfonyl chloride (109 mg, 0.47 mmol) was added in one portion and the mixture was stirred at room temperature for 2 h. Brine (10 mL) was added and the reaction mixture was extracted with ethyl acetate (3 x 20 mL). The combined organic extracts were dried over anhydrous sodium sulfate and concentrated in *vacuo.* The resulting oil was subjected to chromatography on 25 g of silica gel and eluted with 9:1 hexane/ethyl acetate then 7:3 hexane/ethyl acetate to give the title compound as a white solid (135 mg, 64%).

### 4D) (3S,6R)-Bicyclo[4.4.0]-1,4-diaza-3-(3-N,N'-(di-tert-butoxycarbonyl)guanidinopropyl)-4-N-(4-tert-butylbenzenesulfonyl)-2,5-decanedione

Part 4C diketopiperazine (296 mg, 0.44 mmol) was dissolved in 1% trifluoroacetic acid in dichloromethane (30 mL) and stirred for 30 min. The reaction mixture was concentrated *in vacuo* and the resulting oil was subjected to chromatography on 25 g of silica gel, eluted with 1:1 hexane/ethyl acetate then 4:1 dichloromethane/methanol and lyophilized to give (3S,6R)-bicyclo[4.4.0]-1,4-diaza-3-(3-aminopropyl)-4-N-(4-*tert*-butylbenzenesulfonyl)-2,5-decanedione, trifluoroacetate salt as a white solid.

To a solution of the above amine in dichloromethane (10 mL) was added triethylamine (0.061 mL, 0.44 mmol, Aldrich) and N,N'-di-*tert*-butoxy-N"-trifluoromethanesulfonyl guanidine (157 mg, 0.40 mmol, *Journal of Organic Chemistry* 63(12):3804-3805 (1998). After stirring for 12 h, the reaction mixture was poured into dichloromethane (50 mL) and washed with 1M aqueous sodium bisulfate (10 mL), 5% aqueous sodium bicarbonate (10 mL) and water (10 mL). The organic layer was dried over anhydrous sodium sulfate and concentrated *in vacuo.* The resulting oil was subjected to chromatography on 20 g of silica gel and eluted with 9:1 hexane/ethyl acetate then 1:1 hexane/ethyl acetate to give the title compound as a white solid (239 mg, 93%).

### 4E) (3S,6R)-Bicyclo[4.4.0]-1,4-diaza-3-(3-guanidinopropyl)-4-N-(4-tert-butylbenzenesulfonyl)-2,5-decanedione, trifluoroacetate salt

A solution of Part 4D diketopiperazine (100 mg, 0.16 mmol) was dissolved in 1:1 trifluroacetic acid/dichloromethane (2 mL), stirred for 1 h and the reaction mixture was concentrated in vacuo. The resulting oil was subjected to chromatography on 5 g of silica gel, eluted with 19:1 dichloromethane/methanol then 9:1 dichloromethane/methanol and lyophilized to give the title compound as a white solid (88 mg, 96%). Electrospray m.s.: (M+H⁺) at 464.5.

### EXAMPLE 5

### (3S,6R)-Bicyclo[4.4.0]-1,4-diaza-3-(4-guanidinobutyl)-4-N-(4-tert-butylsulfonyl)-2,5-decanedione, trifluroacetate salt

### 5A) 1-[N^{ε}-(4-Methyltrityl) -N^{α}-(9-fluorenylmethoxycarbonyloxy)-L-lysinyl]-D-2-piperidinecarboxylic acid, allyl ester

Example 1 pipecolic ester (253 g, 0.94 mmol) was dissolved in 1:1 trifluroacetic acid/dichloromethane (5 mL) and stirred for 2 h. The reaction mixture was concentrated *in vacuo* and placed on a vacuum pump overnight. The resulting oil was dissolved in dimethylformamide (5 mL), cooled to 0°C and diisopropylethylamine (0.49 mL, 2.82 mmol, Aldrich) was added. After stirring for 5 min, N^{ε}-(4-methyltrityl)-N^{α}-(9-fluorenylmethoxycarbonyloxy)-L-lysine (706 mg, 1.13 mmol, Novabiochem), N-hydroxybenzotriazole (202 mg, 1.32 mmol, Novabiochem) and 2-(1H-benzotriazole-1-yl)-1,1,3,3-tetramethyluronium tetrafluoroborate (424 mg, 1.32 mmol, Novabiochem) were added. The reaction mixture was stirred for 72 h, poured into ethyl acetate (125 mL) and washed with 10% hydrochloric acid (2 x 25 mL), saturated sodium bicarbonate solution (2 x 25 mL) and brine (25 mL). The organic layer was dried over anhydrous sodium sulfate and concentrated *in vacuo*. The resulting oil was subjected to chromatography on 50 g of silica gel; eluted with 3:1 hexane/ethyl acetate to give the title compound as a white solid (605 mg, 83%).

### 5B) (3S,6R)-Bicyclo[4.4.0]-1,4-diaza-3-(4-N-(4-methyltrityl)butyl)-2,5-decanedione

To a solution of Part 5A ester (250 mg, 0.32 mmol) in dichloromethane (64 mL) was added piperidine (1.58 mL, 16.0 mmol, Aldrich) and the reaction mixture was stirred for 2 h. The reaction mixture was concentrated *in vacuo* and the resulting oil was subjected to chromatography on 25 g of silica gel and eluted with ethyl acetate to give the title compound as a white solid (149 mg, 94%).

### 5C) (3S,6R)-Bicyclo[4.4.0]-1,4-diaza-3-(4-N-(4-methyltrityl)butyl)-4-N-(4-tert-butylbenzenesulfonyl)-2,5-decanedione

To a solution of Part 5B diketopiperazine (50 mg, 0.10 mmol) in anhydrous tetrahydrofuran (1.5 mL, Aldrich) under a nitrogen atmosphere at 0°C was added 1.0 M lithium bis(trimethylsilyl)amide in tetrahydrofuran (0.14 mL, 0.14 mmol, Aldrich) and the reaction mixture was stirred for 1 h. 4-tert-Butylbenzenesulfonyl chloride (35 mg, 0.15 mmol) was added in one portion and the mixture was stirred at room temperature for 2 h. Brine (5 mL) was added and the reaction mixture was extracted with ethyl acetate (3 x 10 mL). The combined organic extracts were dried over anhydrous sodium sulfate and concentrated *in vacuo*. The resulting oil was subjected to chromatography on 10 g of silica gel and eluted with 9:1 hexane/ethyl acetate then 7:3 hexane/ethyl acetate to give the title compound as a white solid (47 mg, 68%).

### 5D) (3S,6R)-Bicyclo[4.4.0]-1,4-diaza-3-(4-aminobutyl)-4-N-(4-tert-butylbenzenesulfonyl)-2,5-decanedione, trifluoroacetate salt

Part 5C diketopiperazine (57 mg, 0.082 mmol) was dissolved in 1% trifluoroacetic acid in dichloromethane (2 mL) and stirred for 15 min. The reaction mixture was concentrated *in vacuo* and the resulting oil was subjected to chromatography on 2 g of silica gel, eluted with 1:1 hexane/ethyl acetate then methanol and lyophilized to give the title compound as a white solid (40 mg, 89%).

### 5E) (3S,6R)-Bicyclo[4.4.0]-1,4-diaza-3-(4-N,N'-(di-tert-butoxycarbonyl)guanidinobutyl)-4-N-(4-tert-butylbenzenesulfonyl)-2,5-decanedione

To a solution of Part 5D amine (40 mg, 0.073 mmol) in dichloromethane (5 mL) was added triethylamine (0.011 mL, 0.082 mmol, Aldrich) and N,N'-di-*tert*-butoxy-N"-trifluoromethanesulfonyl guanidine (29 mg, 0.074 mmol, *Journal of Organic Chemistry* 63(12):3804-3805 (1998). After stirring for 12 h, the reaction mixture was poured into dichloromethane (25 mL) and washed with 1M aqueous sodium bisulfate (5 mL), 5% aqueous sodium bicarbonate (5 mL) and water (5 mL). The organic layer was dried over anhydrous sodium sulfate and concentrated *in vacuo.* The resulting oil was subjected to chromatography on 5 g of silica gel and eluted with 9:1 hexane/ethyl acetate then 1:1 hexane/ethyl acetate to give the title compound as a white solid (35 mg, 71%).

### 5F) (3S,6R)-Bicyclo[4.4.0]-1,4-diaza-3-(4-guanidinobutyl)-4-N-(4-tert-butylbenzenesulfonyl)-2,5-decanedione, trifluoroacetate salt

A solution of Part 5E diketopiperazine (35 mg, 0.052 mmol) was dissolved in 1:1 trifluroacetic acid/dichloromethane (1 mL), stirred for 1 h and the reaction mixture was concentrated *in vacuo*. The resulting oil was subjected to chromatography on 4 g of silica gel and eluted with 19:1 dichloromethane/methanol then 9:1 dichloromethane/methanol to give the title compound as a white solid (28 mg, 90%). Electrospray m.s.: (M+H⁺)@ 478.0.

### EXAMPLE 6

### (3S,6R)-Bicyclo[4.4.0]-1,4-diaza-3-(3-guanidinopropyl)-4-N-(3-methyl-8-quinolinesulfonyl)-2,5-decanedione, hydrochloride salt

### 6A) (3S,6R)-Bicyclo[4.4.0]-1,4-diaza-3-(3-N-(4-methyltrityl)aminopropyl)-4-N-(3-methyl-8-quinolinesulfonyl)-2,5-decanedione

To a solution of Example 4 Part B diketopiperazine (350 mg, 0.73 mmol) in anhydrous tetrahydrofuran (12 mL, Aldrich) under a nitrogen atmosphere at 0°C was added 1.0 M lithium bis(trimethylsilyl)amide in tetrahydrofuran (0.88 mL, 0.88 mmol, Aldrich) and the reaction mixture was stirred for 1 h. 3-Methyl-8-quinolinesulfonyl chloride (168 mg, 0.69 mmol) was added in one portion and the mixture was stirred at room temperature for 2 h. Brine (15 mL) was added and the reaction mixture was extracted with ethyl acetate (3 x 25 mL). The combined organic extracts were dried over anhydrous sodium sulfate and concentrated *in vacuo.* The resulting oil was subjected to chromatography on 35 g of silica gel and eluted with 1:1 hexane/ethyl acetate to give the title compound as a white solid (304 mg, 64%).

### 6B) (3S,6R)-Bicyclo[4.4.0]-1,4-diaza-3-(3-aminopropyl)-4-N-(3-methyl-8-quinolinesulfonyl)-2,5-decanedione, trifluoroacetate salt

Part 6A diketopiperazine (304 mg, 0.44 mmol) was dissolved in 1% trifluoroacetic acid in dichloromethane (30 mL) and stirred for 30 min. The reaction mixture was concentrated *in vacuo* and the resulting oil was subjected to chromatography on 25 g of silica gel, eluted with 1:1 hexane/ethyl acetate then 4:1 dichloromethane/methanol and lyophilized to give the title compound as a white solid (240 mg, 100%).

### 6C) (3S,6R)-Bicyclo[4.4.0]-1,4-diaza-3-(3-N,N'-(di-tert-butoxycarbonyl)guanidinopropyl)-4-N-(3-methyl-8-quinolinesulfonyl)-2,5-decanedione

To a solution of Part 6B amine (240 mg, 0.44 mmol) in dichloromethane (10 mL) was added triethylamine (0.12 mL, 0.88 mmol, Aldrich) and N,N'-di-*tert*-butoxy-N"-trifluoromethanesulfonyl guanidine (164 mg, 0.42 mmol, *Journal of Organic Chemistry* 63(12):3804-3805 (1998). After stirring for 12 h, the reaction mixture was poured into dichloromethane (50 mL) and washed with 1M aqueous sodium bisulfate (10 mL), 5% aqueous sodium bicarbonate (10 mL) and water (10 mL). The organic layer was dried over anhydrous sodium sulfate and concentrated *in vacuo.* The resulting oil was subjected to chromatography on 25 g of silica gel and eluted with 1:1 hexane/ethyl acetate to give the title compound as a white solid (216 mg, 73%).

### 6D) (3S,6R)-Bicyclo[4.4.0]-1,4-diaza-3-(3-guanidinopropyl)-4-N-(3-methyl-8-quinolinesulfonyl)-2,5-decanedione, hydrochloride salt

A solution of Part 6C diketopiperazine (10 mg, 0.015 mmol) was dissolved in 3N hydrochloric acid in ethyl acetate (0.27 mL), stirred for 1 h, the reaction mixture was concentrated *in vacuo* and lyophilized to give the title compound as a white solid (7 mg, 88%). Electrospray m.s.: (M+H⁺)@ 473.5.

### EXAMPLE 7

### (3S,6R,8R)-Bicyclo[4.4.0]-1,4-diaza-3-(3-guanidinopropyl)-4-N-(3-methyl-8-quinolinesulfonyl)-8-methyl-2,5-decanedione, hydrochloride salt

### 7A) (2R, 4R) -1- [N^{γ}-(4-Methyltrityl)-N^{α}-(9-fluorenylmethoxycarbonyloxy)-L-ornithinyl]-4-methyl-2-piperidinecarboxylic acid, allyl ester

Example 2 pipecolic ester (406 mg, 1.43 mmol) was dissolved in 1:1 trifluroacetic acid/dichloromethane (7 mL) and stirred for 2 h. The reaction mixture was concentrated *in vacuo* and placed on a vacuum pump overnight. The resulting oil was dissolved in dimethylformamide (7 mL), cooled to 0°C and diisopropylethylamine (0.75 mL, 4.29 mmol, Aldrich) was added. After stirring for 5 min, N^{γ}-(4-methyltrityl)-N^{α}-(9-fluorenylmethoxycarbonyloxy)-L-ornithine (1.05 g, 1.72 mmol, Novabiochem), N-hydroxybenzotriazole (306 mg, 2.00 mmol, Novabiochem) and 2-(1H-benzotriazole-1-yl)-1,1,3,3-tetramethyluronium tetrafluoroborate (642 mg, 2.00 mmol, Novabiochem) were added. The reaction mixture was stirred for 72 h, poured into ethyl acetate (125 mL) and washed with 10% hydrochloric acid (2 x 25 mL), saturated sodium bicarbonate solution (2 x 25 mL) and brine (25 mL). The organic layer was dried over anhydrous sodium sulfate and concentrated *in vacuo*. The resulting oil was subjected to chromatography on 75 g of silica gel and eluted with 3:1 hexane/ethyl acetate to give the title compound as a white solid (1.05 g, 95%).

### 7B) (3S,6R,8R)-Bicyclo[4.4.0]-1,4-diaza-3-(3-N-(4-methyltrityl)aminopropyl)-8-methyl-2,5-decanedione

To a solution of Part 7A ester (958 mg, 1.23 mmol) in dichloromethane (246 mL) was added piperidine (6.1 mL, 61.7 mmol, Aldrich) and the reaction mixture was stirred for 4 h. The reaction mixture was concentrated *in vacuo* and the resulting oil was subjected to chromatography on 65 g of silica gel and eluted with 4:1 hexane/ethyl acetate then 1:1 hexane/ethyl acetate to give the title compound as a white solid (520 mg, 96%).

### 7C) (3S,5R,8R)-Bicyclo[4.4.0]-1,4-diaza-3-(3-N-(9-methyltrityl)aminopropyl)-4-N-(3-methyl-8-quinolinesulfonyl)-8-methyl-2,5-decanedione

To a solution of Part 7B diketopiperazine (250 mg, 0.50 mmol) in anhydrous tetrahydrofuran (7 mL, Aldrich) under a nitrogen atmosphere at 0° C was added 1.0 M lithium bis(trimethylsilyl)amide in tetrahydrofuran (0.50 mL, 0.50 mmol, Aldrich) and the reaction mixture was stirred for 1 h. 3-Methyl-8-quinolinesulfonyl chloride (97 mg, 0.40 mmol) was added in one portion and the mixture was stirred at room temperature for 2 h. Brine (7 mL) was added and the reaction mixture was extracted with ethyl acetate (3 x 20 mL). The combined organic extracts were dried over anhydrous sodium sulfate and concentrated *in vacuo*. The resulting oil was subjected to chromatography on 25 g of silica gel and eluted with 3:1 hexane/ethyl acetate then 3:2 hexane/ethyl acetate to give the title compound as a white solid (177 mg, 63%).

### 7D) (3S,6R,BR)-Bicyclo[4.4.0]-1,4-diaza-3-(3-aminopropyl)-4-N-(3-methyl-8-quinolinesulfonyl)-8-methyl-2,5-decanedione, trifluoroacetate salt

Part 7C diketopiperazine (264 mg, 0.38 mmol) was dissolved in 1% trifluoroacetic acid in dichloromethane (26 mL) and stirred for 30 min. The reaction mixture was concentrated *in vacuo* and the resulting oil was subjected to chromatography on 20 g of silica gel, eluted with 1:1 hexane/ethyl acetate then 19:1 dichloromethane/methanol and lyophilized to give the title compound as a white solid (206 mg, 100%).

### 7E) (3S,6R,8R)-Bicyclo[4.4.0]-1,4-diaza-3-(3-N,N'-(di-tert-butoxycarbonyl)guanidinopropyl)-4-N-(3-methyl-8-quinolinesulfonyl)-8-methyl-2,5-decanedione

To a solution of Part 7D amine (206 mg, 0.38 mmol) in dichloromethane (8 mL) was added triethylamine (0.053 mL, 0.38 mmol, Aldrich) and N,N'-di-tert-butoxy-N"-trifluoromethanesulfonyl guanidine (82 mg, 0.34 mmol, *Journal of Organic Chemistry* 63(12):3804-3805 (1998). After stirring for 12 h, the reaction mixture was poured into dichloromethane (50 mL) and washed with 1M aqueous sodium bisulfate (10 mL), 5% aqueous sodium bicarbonate (10 mL) and water (10 mL). The organic layer was dried over anhydrous sodium sulfate and concentrated *in vacuo.* The resulting oil was subjected to chromatography on 15 g of silica gel and eluted with 1:1 hexane/ethyl acetate to give the title compound as a white solid (140 mg, 60%).

### 7F) (3S,6R,8R)-Bicyclo[4.4.0]-1,4-diaza-3-(3-guanidinopropyl)-4-N-(3-methyl-8-quinolinesulfonyl)-8-methyl-2,5-decanedione, hydrochloride salt

A solution of Part 7E diketopiperazine (10 mg, 0.15 mmol) was dissolved in 3N hydrochloric acid in ethyl acetate (0.27 mL), stirred for 1 h, the reaction mixture was concentrated *in vacuo* and lyophilized to give the title compound as a white solid (7.5 mg, 94%). Electrospray m.s.: (M+H⁺) at 487.5.

### EXAMPLE 8

### (3S,6R,8R)-Bicyclo[4.4.0]-1,4-diaza-3-(3-guanidinopropyl)-4-N-(1,2,3,4-tetrahydro-3-methyl-8-quinolinesulfonyl)-8-methyl-2,5-decanedione, hydrochoride salt

### 8A) (2R,4R)-1-[N^{γ}-(4-Methoxy-2,3,6-trimethyl benzenesulfonyl)-N^{α}-(tert-butoxycarbonyl)-L-arginyl]-4-methyl-2-piperidinecarboxylic acid

Example 2 pipecolic ester (375 mg, 1.32 mmol) was dissolved in 1:1 trifluroacetic acid/dichloromethane (8 mL) and stirred for 2 h. The reaction mixture was concentrated *in vacuo* and placed on a vacuum pump overnight. The resulting oil was dissolved in dimethylformamide (8 mL), cooled to 0°C and diisopropylethylamine (1.15 mL, 6.6 mmol, Aldrich) was added. After stirring for 5 min, N^{γ}-(4-methoxy-2,3,6-trimethylbenznesulfonyl)-N^{α}-(*tert*-butoxycarbonyl)-L-arginine (769 mg, 1.58 mmol, Novabiochem), N-hydroxybenzotriazole (283 mg, 1.85 mmol, Novabiochem) and 2-(1H-benzotriazole-1-yl)-1,1,3,3-tetramethyluronium (594 mg, 1.85 mmol, Novabiochem) were added. The reaction mixture was stirred for 18 h, poured into ethyl acetate (75 mL) and washed with 10% citric acid (2 x 10 mL), saturated sodium bicarbonate solution (2 x 10 mL) and brine (10 mL). The organic layer was dried over anhydrous sodium sulfate and concentrated in vacuo to give (2R,4R)-1-[N^{γ}-(4-methoxy-2,3,6-trimethylbenzenesulfonyl)-N^{α}-(*tert*-butoxycarbonyl)-L-arginyl]-4-methyl-2-piperidinecarboxylic acid, allyl ester as a white foamy solid.

The peptide from above was dissolved in 1:1 trifluoroacetic acid/dichloromethane (8 mL) and stirred for 5 min. The reaction mixture was concentrated *in vacuo* and placed on a vacuum pump for 5 min. The resulting oil was dissolved in dichloromethane (20 mL) and triethylamine (1.8 mL, 13.2 mmol, Aldrich) and 3-methyl-8-quinolinesulfonyl chloride (319 mg, 1.32 mmol) were added. After stirring for 1 h, the reaction mixture was poured into dichloromethane (50 mL) and washed with water (15 mL) and brine (15 mL). The organic layer was dried over anhydrous sodium sulfate and concentrated *in vacuo* to give (2R,4R)-1-[N^{γ}-(4-methoxy-2,3,6-trimethylbenzenesulfonyl)-N^{α}-(3-methyl-8-quinolinesulfonyl)-L-arginyl]-4-methyl-2-piperidinecarboxylic acid, allyl ester as a pale yellow foamy solid.

The peptide from above was dissolved in absolute ethanol (14 mL) and 1N aqueous sodium hydroxide (3.6 mL). After stirring for 21 h, the reaction mixture was adjusted to pH 7 with 1N hydrochloric acid and concentrated *in vacuo*. The resulting residue was dissolved in 1:1 ethyl acetate/water (20 mL), the solution was adjusted to pH 11 with 1N sodium hydroxide and extracted with ethyl acetate (2x30 mL). The aqueous layer was adjusted to pH 2 with 1N hydrochloric acid and extracted with chloroform (3 x 50 mL). The combined chloroform extracts were dried over anhydrous sodium sulfate and concentrated *in vacuo* to give the title compound as a white foamy solid (829 mg, 88% over 3 steps).

### 8B) (2R, 4R) -1- [N^{γ}-(4-Methoxy-2,3,6-trimethylbenzenesulfonyl)-N^{α}-(1,2,3,4-tetrahydro-3-methyl-8-quinolinesulfonyl)-L-arginyl]-4-methyl-2-piperidinecarboxylic acid

A suspension of Part 8A acid (100 mg, 0.14 mmol) and 10% palladium on carbon (28 mg, Aldrich) in 95% ethanol (2 mL) and 1N hydrochloric acid (0.12 mL) was heated in a 15 mL sealed tube under a hydrogen atmosphere at 75°-80°C for 65 h. The mixture was cooled to room temperature, filtered and concentrated in vacuo. The resulting oil was subjected to chromatography on 15 g of silica gel and eluted with ethyl acetate then 4:1 dichloromethane/methanol to give the title compound as a white solid (58 mg, 58%).

### 8C) (3S,6R,8R)-bicyclo[4.4.0]-1,4-diaza-3-(3-(N^{γ}-4-methoxy-2,3,6-rimethylbenzenesulfonyl)guanidinopropyl)-4-N-(3-methyl-1,2,3,4-tetrahydro-8-quinolinesulfonyl)-8-methyl-2,5-decanedione

To a solution of Part 8B acid (58 mg, 0.080 mmol) in dichloromethane (16 mL) was added N-hydroxybenzotriazole (12 mg, 0.080 mmol, Novabiochem), 2-(1H-benzotriazole-1-yl)-1,1,3,3-tetramethyluronium tetrafluoroborate (26 mg, 0.080 mmol, Novabiochem) and diisopropylethylamine (0.014 mL, 0.080 mmol, Aldrich). The reaction mixture was stirred for 3 h, poured into ethyl acetate (30 mL) and washed with saturated sodium bicarbonate solution (10 mL) and brine (10 mL). The organic layer was dried over anhydrous sodium sulfate and concentrated in vacuo. The resulting oil was subjected to chromatography on 6 g of silica gel, eluted with 1:4 hexane/ethyl acetate and lyophilized to give the title compound as a white solid (51 mg, 91%).

### 8D) (3S,6R,8R)-Bicyclo[4.4.0]-1,4-diaza-3-(3-guanidinopropyl)-4-N-(1,2,3,4-tetrahydro-3-methyl-8-quinolinesulfonyl)-8-methyl-2,5-decanedione, hydrochoride salt

Part 8C diketopiperazine (36 mg, 0.051 mmol) was dissolved in 1:1 trifluoroacetic acid/dichloromethane (4 mL), stirred for 20 h and concentrated *in vacuo.* The resulting oil was dissolved in 3N hydrochloric acid in ethyl acetate (4 mL), stirred for 1 h and concentrated *in vacuo.* The resulting oil was subjected to chromatography on 2 g of silica gel, eluted with ethyl acetate then methanol and lyophilized to give the title compound as a white solid (20 mg, 74%). Electrospray m.s.: (M+H⁺) at 491.5.

### EXAMPLE 9

### Biological Assays of Cycloargatroban (Formula I) where R¹, R² and R⁴ are hydrogen, R³ is Me=CH₃, R⁵ is 1,2,3,4-tetrahydro-3-methyl-8-quinolinesulfonyl and R⁶ is 3-guanidinopropyl

The activity and selectivity of the present invention can be identified by determination of the inhibition constant (Ki) for serine proteases such thrombin and trypsin and fibrinolytic enzymes such as urokinase, plasmin and tissue plasminogen activator (tPA). All of enzymes are purchased from Sigma. The general assay conditions are as follows. The fluorogenic substrates are dissolved in DMSO and diluted using assay buffer containing 50 mM Tris·HCl (pH 7.8 at 25°C), 0.1 M NaCl and 0.1% polyethylene glycol 8000 (PEG 8000). The fluorogenic substrates are Tos-Gly-Pro-Arg-AMC (Sigma, Km = 4.0 µM at 25°C, pH7.8)(Yudu Cheng et al., *Biochemistry,* 1996, 35: 13021-13029) for thrombin, Bz-Arg-AMC·HCl (Bachem, Km=59±2 µM at 25°C, pH8.0) for trypsin, N-Cbz-Gly-Gly-Arg-AMC (Sigma, Kₘ = 400 µM at 24 °C and pH 7.5) for urokinase, D-Ala-Leu-Lys-AMC (Sigma, Kₘ = 620 µM at 25°C and pH 8.0) for plasmin and Boc-L-(p-F) FPR-ANSNH-C₂H₅ (Haematologic Technologies Inc., Kₘ = 71 µM at 25°C and pH 7.4) for tPA. The assays were conducted using Hitachi F2500 spectrophotometer under ambient temperature and at the excitation and emission wavelengths of 383nm and 455nm, respectively. The typical progressive data of the enzymatic assays are shown in Figs. 1-3, and the determination of inhibition constant (Ki) is shown in Fig. 4. The assay results, in comparison to argatroban, an anticoagulant currently in clinic use and with the chemical structure XIII, are shown in Table I.

The results demonstrate that the cycloargatroban derived from the cyclization of backbone of argatroban are featured by: (1) Retaining high thrombin inhibition activity (2.1-fold lower than argatroban); (2) Achieving high selectivity for thrombin over trypsin (12-fold higher than argatroban); (3) Retaining no significant inhibition for fibrinolytic enzymes (similar to argatroban); (4) Retaining the diversity in side chains (similar to argatroban).

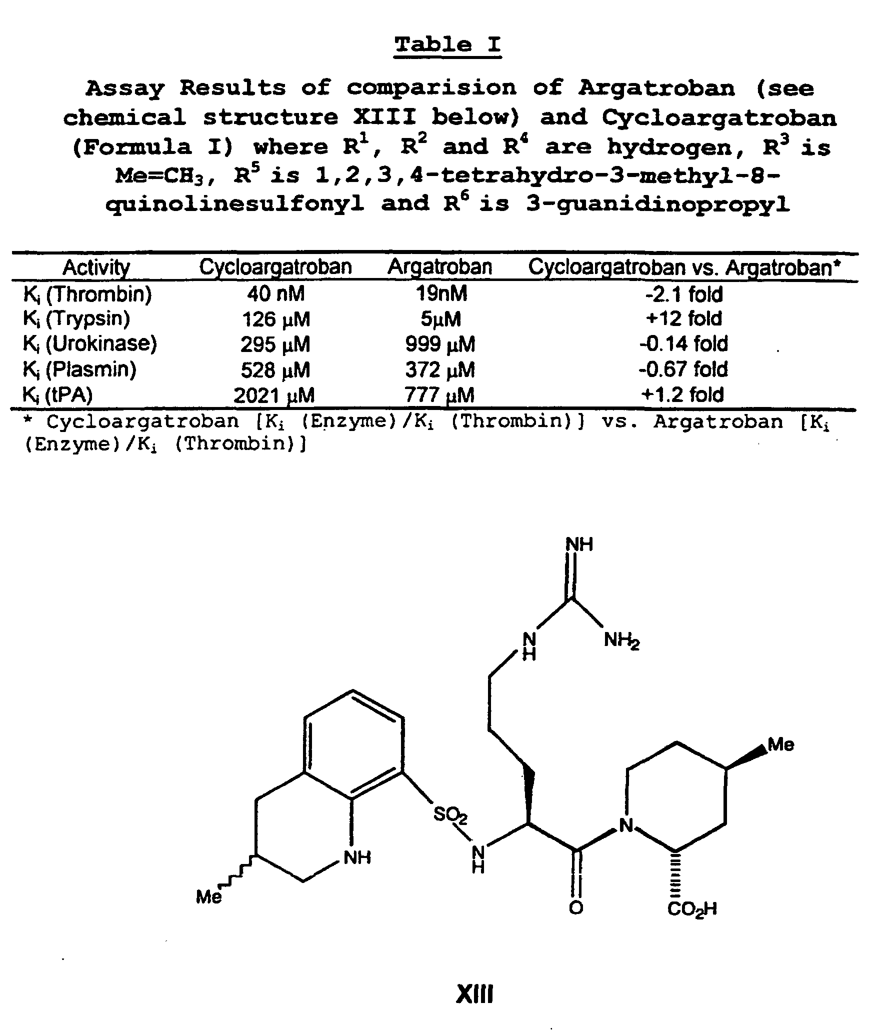

### EXAMPLE 10

### Ex vivo Coagulation Assay

The *ex vivo* anticoagulant effects of NPI999 in comparison with argatroban, a reference anticoagulant currently in clinical use with the following chemical structure: 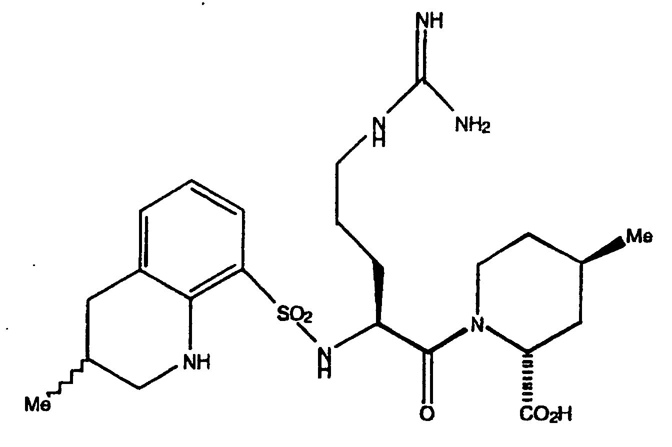 were determined by measuring the prolongation of the activated partial thromboplastin time (APTT) over a broad concentration range of each added thrombin inhibitor, using pooled normal human plasma. Frozen-pooled human plasma was obtained from Sigma. Measurement of APTT was made using the ELECTRA™ 800 automated coagulometer (Medical Laboratory Automation Inc.) using the automated APTT reagent (SIGMA) as the initiator of clotting according to the manufacture's instructions. The assay was conducted by making a series of dilution of the reference and test compounds in rapidly thawed plasma (compound: plasma = 0.1 ml:0.9ml) followed by adding the mixed solution to the wells of the assay carousel. Tris buffers (pH 7.8 at 25°C) were used through the entire assay.

Fig. 5 depicts the effect of NPI999 (open circle) and argatroban (open square) on the activated partial thromboplastin time (APTT) of normal citrated human plasma. As shown in Fig. 5, both compounds prolonged the APTT in a dose dependent manner. This demonstrates the deactivation of coagulating enzymes presented in the human plasma. It is to be noted that APTT measures the overall anticoagulant effects of a compound against the clotting enzymes such as thrombin, plasmin, urokinase, tissue plasminogen activator (tPA) and serine protease such as trypsin, factor X etc. Therefore, the less strong effect of cycloargatroban (formula I) than argatroban on APTT may be attributed to higher selectivity of cycloargatroban (formula I) to the clotting and serine protease than argatroban.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A compound of the following structure I: 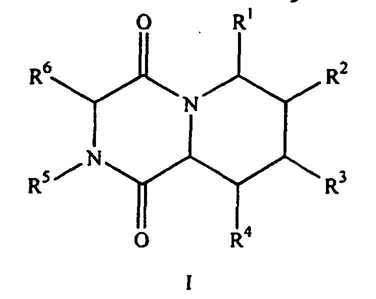 or a pharmaceutically acceptable salt and stereoisomer thereof, wherein wherein R¹, R² and R⁴ consist of a hydrogen, alkyl or aryl moiety, R³ consist of an alkyl or aryl moiety, R⁵ consists of a hydrogen, alkyl, aryl, hydroaryl, heteroaryl, hydroheteroaryl, sulfonylalkyl, sulfonylaryl, sulfonylhydroaryl, sulfonylheteroaryl or sulfonylhydroheteroaryl moiety, and R⁶ consists of a hydrogen, alkyl, aryl, hydroaryl, heteroaryl or hydroheteroaryl moiety.

2. A compound according to claim 1, wherein wherein R¹, R² and R⁴ consist of a hydrogen, alkyl or aryl moiety, R³ consist of an alkyl or aryl moiety, and wherein R⁵ consists of an alkyl, aryl, hydroaryl, heteroaryl or hydroheteroaryl moiety.

3. A compound according to claim 2, wherein R³ consists of a methyl moiety, R⁵ consists of 1,2,3,4-tetrahydro-3-methyl-8-quinolinesulfonyl, and R⁶ consists of 3-guanidinopropyl.

4. A pharmaceutical composition comprising a compound according to claim 1 as an active ingredient in association with a pharmaceutically acceptable carrier.

5. A pharmaceutical composition comprising a compound according to claim 1 in association with a pharmaceutically acceptable carrier, said pharmaceutical composition being suitable for oral administration.

6. A use of the compound according to claim 1 for the manufacture of a medicament for substantially preventing thrombin activity in a mammal or a human or a tissue thereof.

7. A use of the compound according to claim 1 for the manufacture of a medicament for treating a coagulation disorder in a mammal or a human or a tissue thereof.

8. The use according to claim 6 or 7, wherein the disorder consists of thrombosis or heparin-induced thrombocytopenia (HIT).

9. A use of the compound according to claim 1 for the manufacture of a medicament for substantially preventing fibrinolytic enzyme activity in a mammal or a human or a tissue thereof.

10. The use according to claim 9, wherein said fibrinolytic enzyme is selected from the group of urokinase, plasmin and tissue plasminogen activator (tPA).

## Patentansprüche

1. Verbindung der folgenden Struktur I: 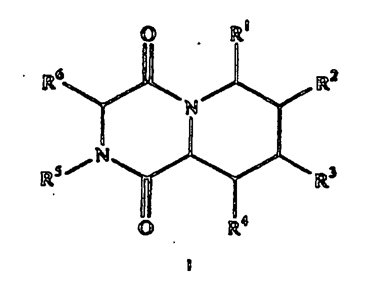 oder ein pharmazeutisch annehmbares Salz und Stereoisomer hiervon, wobei R¹, R² und R⁴ aus einem Wasserstoffatom, Alkyl- oder Arylrest bestehen, R³ aus einem Alkyl- oder Arylrest besteht, R⁵ aus einem Wasserstoffatom, Alkyl-, Aryl-, Hydroaryl-, Heteroaryl-, Hydroheteroaryl-, Sulfonylalkyl-, Sulfonylaryl-, Sulfonylhydroaryl-, Sulfonylheteroaryl- oder Sulfonylhydroheteroarylrest besteht und R⁶ aus einem Wasserstoffatom, Alkyl-, Aryl-, Hydroaryl-, Heteroaryl- oder Hydroheteroarylrest besteht.

2. Verbindung nach Anspruch 1, wobei R¹, R² und R⁴ aus einem Wasserstoffatom, Alkyl- oder Arylrest bestehen, R³ aus einem Alkyl- oder Arylrest besteht und wobei R⁵ aus einem Alkyl-, Aryl-, Hydroaryl-, Heteroaryl- oder Hydroheteroarylrest besteht.

3. Verbindung nach Anspruch 2, wobei R³ aus einem Methylrest besteht, R⁵ aus 1,2,3,4-Tetrahydro-3-methyl-8-chinolinsulfonyl besteht und R⁶ aus 3-Guanidinopropyl besteht.

4. Pharmazeutische Zusammensetzung, umfassend eine Verbindung nach Anspruch 1 als aktiven Bestandteil in Verbindung mit einem pharmazeutisch annehmbaren Träger.

5. Pharmazeutische Zusammensetzung, umfassend eine Verbindung nach Anspruch 1 in Verbindung mit einem pharmazeutisch annehmbaren Träger, wobei die pharmazeutische Zusammensetzung für die orale Verabreichung geeignet ist.

6. Verwendung der Verbindung nach Anspruch 1 für die Herstellung eines Medikaments, um im Wesentlichen die Thrombin-Aktivität bei einem Säuger oder einem Menschen oder einem Gewebe hiervon zu verhindern.

7. Verwendung der Verbindung nach Anspruch 1 für die Herstellung eines Medikaments zur Behandlung einer Gerinnungsstörung bei einem Säuger oder einem Menschen oder einem Gewebe hiervon.

8. Verwendung nach Anspruch 6 oder 7, wobei die Störung in einer Trombose- oder Heparin-induzierten Thrombozytopenie (HIT) besteht.

9. Verwendung der Verbindung nach Anspruch 1 für die Herstellung eines Medikaments, um im Wesentlichen die fibrinolytische Enzymaktivität bei einem Säuger oder einem Menschen oder einem Gewebe hiervon zu verhindern.

10. Verwendung nach Anspruch 9, wobei das fibrinolytische Enzym gewählt ist aus der Gruppe von Urokinase, Plasmin und Gewebe-Plasminogen-Aktivator (tPA).

## Revendications

1. Composé répondant à la structure suivante I : 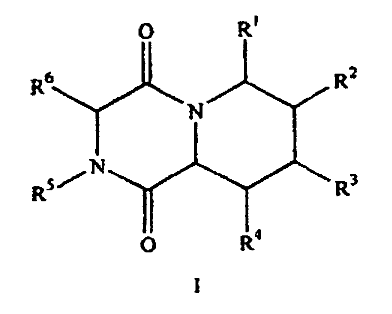 ou un sel et un stéréoisomère pharmaceutiquement acceptables de celui-ci, dans lequel R¹, R² et R⁴ représentent un atome d'hydrogène, un groupe alkyle ou aryle, R³ représente un groupe alkyle ou aryle, R⁵ représente un atome d'hydrogène, un groupe alkyle, aryle, hydroaryle, hétéroaryle, hydrohétéroaryle, sulfonylalkyle, sulfonylaryle, sulfonylhydroaryle, sulfonylhétéroaryle ou sulfonylhydrohétéroaryle, et R⁶ représente un atome d'hydrogène, un groupe alkyle, aryle, hydroaryle, hétéroaryle ou hydrohéteroaryle.

2. Composé selon la revendication 1, dans lequel R¹, R² et R⁴ représentent un atome d'hydrogène, un groupe alkyle ou aryle, R³ représente un groupe alkyle ou aryle et dans lequel R⁵ représente un groupe alkyle, aryle, hydroaryle, hétéroaryle ou hydrohéteroaryle.

3. Composé selon la revendication 2, dans lequel R³ représente un groupe méthyle, R⁵ comprenant 1,2,3,4-tétrahydro-3-méthyl-8-quinolinesulfonyle, et R⁶ comprenant 3-guanidinopropyle.

4. Composition pharmaceutique comprenant un composé selon la revendication 1 en tant que principe actif en association avec un transporteur pharmaceutiquement acceptable.

5. Composition pharmaceutique comprenant un composé selon la revendication 1 en association avec un transporteur pharmaceutiquement acceptable, ladite composition pharmaceutique étant adaptée pour une administration orale.

6. Utilisation du composé selon la revendication 1 pour la fabrication d'un médicament destiné à prévenir de façon substantielle l'activité de la thrombine chez un mammifère ou un être humain ou un de leurs tissus.

7. Utilisation du composé selon la revendication 1 pour la fabrication d'un médicament destiné à traiter un trouble de la coagulation chez un mammifère ou un être humain ou un de leurs tissus.

8. Utilisation selon la revendication 6 ou 7, dans laquelle le trouble correspond à une thrombose où à une thrombocytopénie induite par l'héparine (HIT).

9. Utilisation du composé selon la revendication 1 pour la fabrication d'un médicament destiné à prévenir de façon substantielle l'activité de l'enzyme fibrinolytique chez un mammifère ou un être humain ou un de leurs tissus.

10. Utilisation selon la revendication 9, dans laquelle ladite enzyme fibrinolytique est choisie dans le groupe constitué de l'urokinase, de la plasmine et de l'activateur tissulaire du plasminogène (tPA).
